# EUROPEAN PATENT APPLICATION

(11) **EP 4 400 027 A1**
(43) Date of publication of application: **17.07.2024**
(21) Application number: 22866613.7
(22) Date of filing: 06.09.2022
(51) Int. Cl.: A61B 1/00

(54) **MEDICAL ENDOSCOPE SYSTEM**

(30) Priority: 07.09.2021 US 202163241175 P
(71) Applicant: Chen, Chieh-Hsiao, Taichung City, Taiwan 406 (CN)
(72) Inventor: Chen, Chieh-Hsiao, Taichung City, Taiwan 406 (CN)
(74) Representative: Kretschmann, Dennis
(86) International application number: PCT/CN2022/117377
(87) International publication number: WO 2023/036150

(57) **Abstract**

The present disclosure provides a medical endoscope system, comprising: a sheath device having an end portion; and an optical sensing device disposed to extend within the sheath device and fixed to the end portion. The optical sensing device has a transmission portion and transmits an optical signal received by the optical sensing device through the transmission portion.

## Description

### BACKGROUND OF THE INVENTION

### FIELD OF THE INVENTION

The present disclosure relates to a medical endoscope system and, more particularly, to a medical endoscope system with a simplified operating method, so that a user can be free from the burden of holding a sheath device and an optical sensing device of the medical endoscope system with separate hands at the same time.

### DESCRIPTION OF THE PRIOR ART

In a conventional medical endoscope system, although an optical sensing device (i.e., endoscope device) is disposed to extend within a sheath device, the two devices are actually separate, and the optical sensing device is not fixed to the sheath device. The conventional arrangement of the medical endoscope system thus requires a user (for example, a medical professional) to separately hold and operate the optical sensing device and the sheath device with two hands. During surgery, the user has to hold and operate the sheath device with one hand, while using the other hand to hold and operate the optical sensing device disposed within the sheath device. Such operating method greatly increases the difficulty for users to operate a conventional medical endoscope system. It is difficult for the user to precisely control the sheath device and other medical devices in the sheath device altogether while observing through the optical sensing device. In view of this, it is necessary to provide a medical endoscope system with a simplified operating method, so that the user can be free from the burden of holding a sheath device and an optical sensing device of the medical endoscope system with separate hands at the same time.

### SUMMARY OF THE INVENTION

To remedy the aforesaid drawbacks of the prior art, it is an objective of the present disclosure to provide a medical endoscope system with a simplified operating method, so that the user can be free from the burden of holding a sheath device and an optical sensing device of the medical endoscope system with separate hands at the same time.

Based on the foregoing idea, the present disclosure provides a medical endoscope system, comprising: a sheath device having an end portion; and an optical sensing device disposed to extend within the sheath device and fixed to the end portion, wherein the optical sensing device has a transmission portion and transmits an optical signal received by the optical sensing device through the transmission portion.

In a preferred embodiment of the present disclosure, the end portion of the sheath device is a transparent tubular element.

In a preferred embodiment of the present disclosure, the medical endoscope system further comprises: a liquid passage portion disposed to extend within the sheath device and having a passage port, wherein the passage port of the liquid passage portion is disposed at the end portion of the sheath device, and the medical endoscope system allows a liquid to flow out of the end portion along the liquid passage portion.

In a preferred embodiment of the present disclosure, the optical sensing device has an optical sensing portion, and the medical endoscope system allows the liquid to flow to the end portion to come into contact with the optical sensing portion.

In a preferred embodiment of the present disclosure, the optical sensing portion is a CMOS sensor.

In a preferred embodiment of the present disclosure, the optical sensing device has an illumination portion disposed around the optical sensing portion.

In a preferred embodiment of the present disclosure, the optical sensing device comprises: a first optical sensing portion disposed at the end portion and facing a first direction to receive a first optical signal; and a second optical sensing portion disposed at the end portion and facing a second direction to receive a second optical signal, wherein the first direction is not parallel to the second direction.

In a preferred embodiment of the present disclosure, the sheath device has a first fixing mechanism disposed at a control end portion of the sheath device; the optical sensing device has a second fixing mechanism, and the optical sensing device is fixed to the end portion by causing the second fixing mechanism to be connected and fixed to the first fixing mechanism; and the second fixing mechanism is detachably connected and fixed to the first fixing mechanism.

In a preferred embodiment of the present disclosure, the sheath device has a fixing portion disposed at the end portion, and the optical sensing device is detachably fixed to the fixing portion.

In a preferred embodiment of the present disclosure, the fixing portion is a magnet.

In a preferred embodiment of the present disclosure, the sheath device has a groove disposed to extend on an inner wall surface of the sheath device, wherein the optical sensing portion is connected to the groove and is movable along the groove.

In a preferred embodiment of the present disclosure, the end portion comprises an open end portion, and a retraction distance is provided between the optical sensing device and the open end portion.

In a preferred embodiment of the present disclosure, the medical endoscope system further comprises an electrocautery device fixed to an outside surface of the end portion.

In a preferred embodiment of the present disclosure, the medical endoscope system further comprises an electrocautery device movably disposed to extend within the sheath device, wherein the electrocautery device has a fixing mechanism and an electrocautery portion, and the electrocautery device is detachably fixed to the end portion of the sheath device through the fixing mechanism, so that the electrocautery portion is exposed outside the sheath device.

In a preferred embodiment of the present disclosure, the medical endoscope system further comprises a shutter device movably disposed to extend within the sheath device, wherein the shutter device has a shutter portion, and the shutter device shuts an opening portion of the end portion through the shutter portion.

In a preferred embodiment of the present disclosure, the shutter portion is an inflatable element.

In a preferred embodiment of the present disclosure, the medical endoscope system further comprises a surgical device disposed within the sheath device.

The various aspects of the present disclosure stated above, together with other aspects of the present disclosure, will be clarified further hereinafter based on the following detailed description of the non-restrictive embodiments and their accompanying drawings.

### BRIEF DESCRIPTION OF THE DRAWINGS

FIG. 1A is a schematic view showing the structure of a medical endoscope system according to an embodiment of the present disclosure.
FIG. 1B is a schematic view of the medical endoscope system according to an embodiment of the present disclosure.
FIG. 2 is a schematic view of the medical endoscope system according to an embodiment of the present disclosure.
FIG. 3 is a schematic view of the medical endoscope system according to an embodiment of the present disclosure.
FIG. 4A is a schematic view of the medical endoscope system according to an embodiment of the present disclosure.
FIG. 4B is a schematic view of the medical endoscope system according to an embodiment of the present disclosure.
FIG. 5A is a schematic view of the medical endoscope system according to an embodiment of the present disclosure.
FIG. 5B is a schematic view of the medical endoscope system according to an embodiment of the present disclosure.
FIG. 6 is a schematic view of the medical endoscope system according to an embodiment of the present disclosure.
FIG. 7 is a schematic view of the medical endoscope system according to an embodiment of the present disclosure.
FIG. 8A is a schematic view of the medical endoscope system according to an embodiment of the present disclosure.
FIG. 8B is a schematic view of the medical endoscope system according to an embodiment of the present disclosure.

### DETAILED DESCRIPTION OF THE EMBODIMENTS

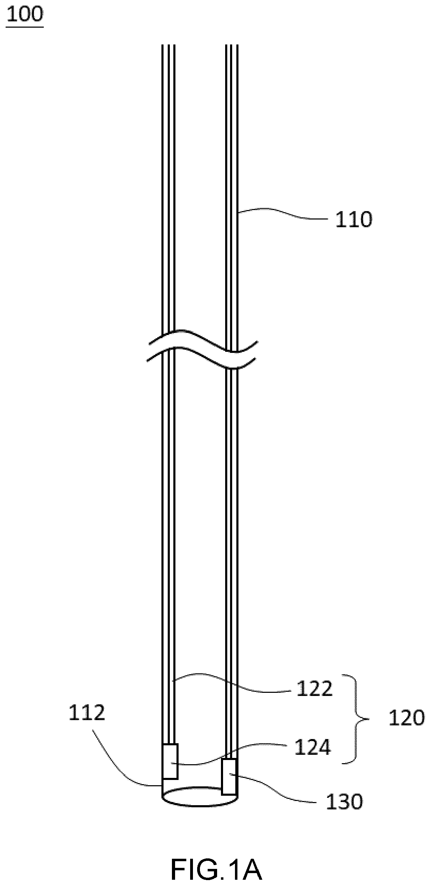

FIG. 1A is a schematic view showing the structure of a medical endoscope system according to an embodiment of the present disclosure. In the embodiment illustrated in FIG. 1A, a medical endoscope system 100 comprises a sheath device 110 and an optical sensing device 120. The sheath device 110 has an end portion 112, and the optical sensing device 120 comprises a transmission portion 122 and an optical sensing portion 124. The optical sensing device 120 is disposed to extend within the sheath device 110 (the description "disposed to extend" means that the optical sensing device 120 extends within the sheath device 110 along the length direction of the sheath device 110; for example, the optical sensing portion 124 of the optical sensing device 120 can be disposed at the end portion 112 of the sheath device 110, while the transmission portion 122 of the optical sensing device 120 can extend from the optical sensing portion 124 to the other end of the sheath device 110 along the internal portion of the sheath device 110), and the optical sensing device 120 is fixed to the end portion 112. Thus, a user (for example, a medical professional) can hold and operate the medical endoscope system 100 at the same time, without having to hold or operate the sheath device 110 and the optical sensing device 120 with two separate hands; as a result, the difficulty in operating the medical endoscope system 10 can be significantly reduced. In one embodiment, another surgical device 130 (including, for example, a medical clamping device or a medical cutting device, but not limited thereto) is disposed within the sheath device 110 to perform medical treatment (including, for example, a minor surgery, but not limited thereto) on a target lesion in the human body. In particular, the surgical device 130 is connected to the sheath device 110 and/or fixed within it as needed, or the surgical device 130 can be movably disposed within the sheath device 110 as needed. Preferably, in the medical endoscope system 110, the optical sensing portion 124 of the optical sensing device 120 is fixed to the end portion 112 of the sheath device 110. The optical sensing device 120 can receive an optical signal through the optical sensing portion 124, and can transmit the optical signal through the transmission portion 122. For example, when the end portion 112 of the sheath device 110 is placed at a target lesion in the human body, the optical sensing portion 124 at the end portion 112 can receive (or detect) an optical signal of the target lesion, and then transmit the optical signal to a display device outside the human body. As a result, the user can observe the images of the target lesion displayed on the display device. Preferably, the end portion 112 of the sheath device 110 can be a transparent tubular element. For example, the sheath device 110 is a transparent tubular device. As a result, the problem that the sheath device 110 may block the field of view of the optical sensing portion 124 can be avoided. In one embodiment, the optical sensing portion 124 of the optical sensing device 120 is a CMOS (complementary metal oxide semiconductor) sensor. Nevertheless, it should be understood that the optical sensing portion 124 of the optical sensing device 120 is not limited to a CMOS sensor, and it can be a sensor of other types as needed. In one embodiment, the optical sensing device 120 can have an illumination portion connected to the optical sensing portion 124 and provide illumination for the view of the surgical field in front of the optical sensing portion 124. The illumination portion can be, for example, an LED (light-emitting diode) device. Preferably, the illumination portion is disposed around the optical sensing portion 124.

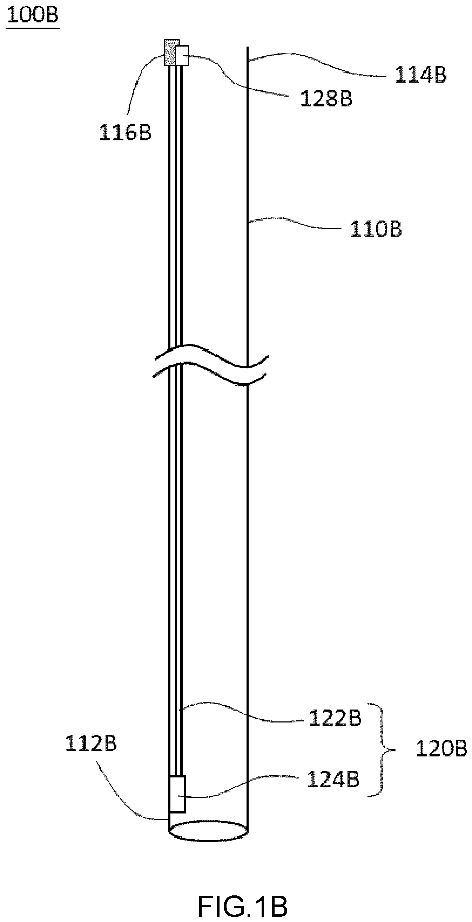

Referring to FIG. 1B, there is shown a schematic view of the medical endoscope system according to an embodiment of the present disclosure. In the embodiment illustrated in FIG. 1B, a medical endoscope system 100B comprises a sheath device 110B and an optical sensing device 120B. The sheath device 110B has an end portion 112B, a control end portion 114B and a first fixing mechanism 116B, and the first fixing mechanism 116B is disposed at the control end portion 114B of the sheath device 110B. The optical sensing device 120B comprises a second fixing mechanism 128B, a transmission portion 122B and an optical sensing portion (may be referred to as the "primary optical sensing portion") 124B. The second fixing mechanism 128B is connected to the transmission portion 122B, and the transmission portion 122B is connected to the optical sensing portion 124B. The optical sensing device 120B is disposed to extend within the sheath device 110B, (the description "disposed to extend" means that the optical sensing device 120B extends within the sheath device 110B along the length direction of the sheath device 110B; for example, the optical sensing portion 124B of the optical sensing device 120B can be disposed at the end portion 112B of the sheath device 110B, while the transmission portion 122B of the optical sensing device 120B can extend from the optical sensing portion 124B to the control end portion 114B of the sheath device 110B). Preferably, the second fixing mechanism 128B of the optical sensing device 120B is connected and fixed to the first fixing mechanism 116B, and thus the optical sensing device 120B is fixed to the end portion 112B of the sheath device 110B. Therefore, the user (for example, a medical professional) can hold and operate the medical endoscope system 100B at the same time, without having to hold or operate the sheath device 110B and the optical sensing device 120B with two separate hands; the difficulty in operating the medical endoscope system 100B can thus be significantly reduced.

It should be understood that the first fixing mechanism 116B and the second fixing mechanism 128B can be, including but not limited to, a snap-engaging mechanism, engaging mechanism, screwing mechanism, binding mechanism, or adhesion mechanism as needed. Therefore, the second fixing mechanism 128B can be connected and fixed to the first fixing mechanism 116B by means of snap-engagement, engagement, screwing, binding, or adhesion etc. as needed. Preferably, the second fixing mechanism 128B is detachably connected and fixed to the first fixing mechanism 116B. Preferably, the optical sensing device 120B being fixed to the end portion 112B of the sheath device 110B, as stated above, shall mean that the optical sensing portion 124B of the optical sensing device 120B is fixed to the end portion 112B of the sheath device 110B. Preferably, the optical sensing device 120B being fixed to the end portion 112B of the sheath device 110B shall mean that the optical sensing device 120B (or the optical sensing portion 124B of the optical sensing device 120B) is fixed to the end portion 112B of the sheath device 110B, wherein the optical sensing device 120B (or the optical sensing portion 124B of the optical sensing device 120B) is not necessarily connected to the end portion 112B of the sheath device 110B directly. Preferably, the external portion (or housing) of the transmission portion 122B is made of a hard material. As a result, regardless of whether the optical sensing portion 124B is connected to the end portion 112B (for example, through a mechanism such as a magnet, to be explained in details), when the second fixing mechanism 128B of the optical sensing device 120B is connected and fixed to the first fixing mechanism 116B, the optical sensing portion 124B of the optical sensing device 120B remains driven by the transmission portion 122B and thus fixed to the end portion 112B of the sheath device 110B).

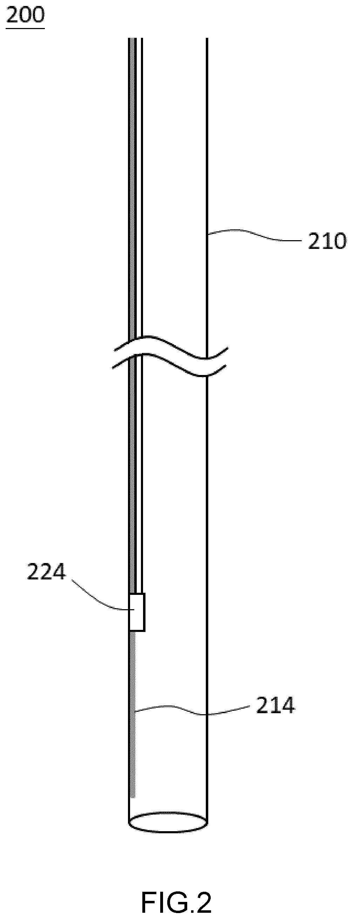

Referring to FIG. 2, there is shown a schematic view of the medical endoscope system according to an embodiment of the present disclosure. In the embodiment illustrated in FIG. 2, a sheath device 210 of a medical endoscope system 200 has a groove 214, and the groove 214 is disposed to extend on an inner wall surface of the sheath device 210 (the description "disposed to extend" means that the groove 214 extends within the sheath device 210 along the length direction of the sheath device 210), wherein the optical sensing portion 224 is connected to the groove 214 and is movable (or slidable) along the groove 214. As a result, the user can place the optical sensing device of the medical endoscope system 200 into the sheath device 210 along the groove 214 as needed, or can remove the optical sensing device from the sheath device 210 along the groove 214 as needed.

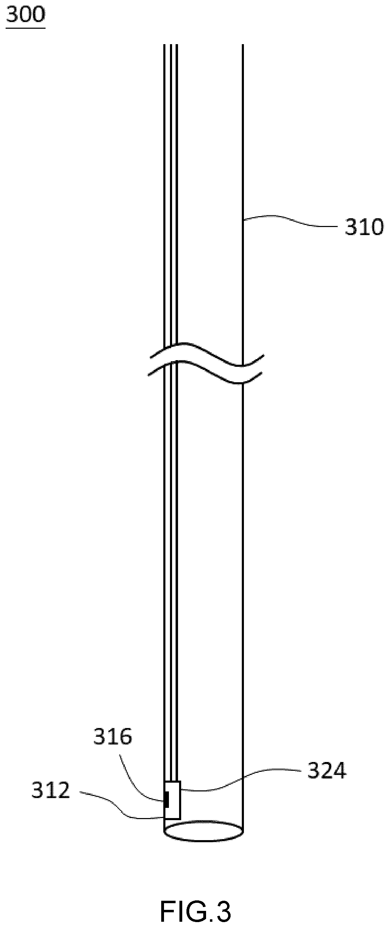

In one embodiment, the sheath device can have a fixing portion, and the fixing portion is disposed at the end portion of the sheath device, wherein the optical sensing device is detachably fixed to the fixing portion. For example, referring to FIG. 3, there is shown a schematic view of the medical endoscope system according to an embodiment of the present disclosure. In the embodiment illustrated in FIG. 3, a sheath device 310 of a medical endoscope system 300 has a fixing portion 316, and the fixing portion 316 is disposed on the inner wall surface at an end portion 312 of the sheath device 310. As a result, the optical sensing device of the medical endoscope system 300 can be fixed to the end portion 312 by the user as needed, or can be removed from the optical sensing device from the end portion 312 as needed. Preferably, the fixing portion 316 is a magnet, and the fixing portion 316 and an optical sensing portion 324 of the optical sensing device can be detachably fixed to each other by a magnetic force, so that the optical sensing portion 324 of the optical sensing device is fixed to the end portion 312.

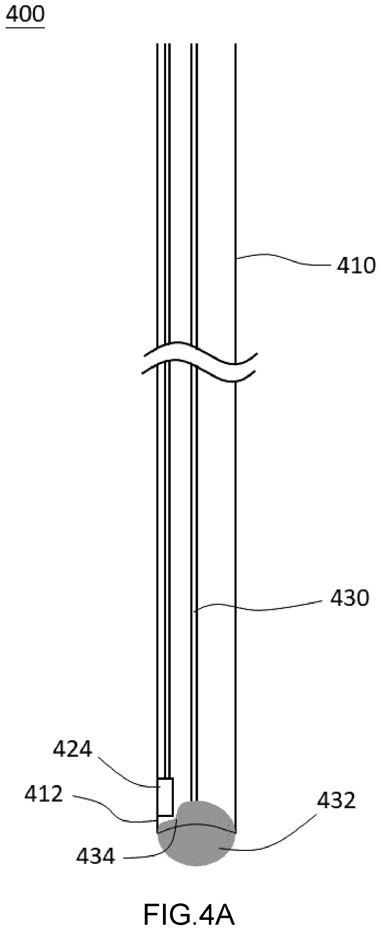

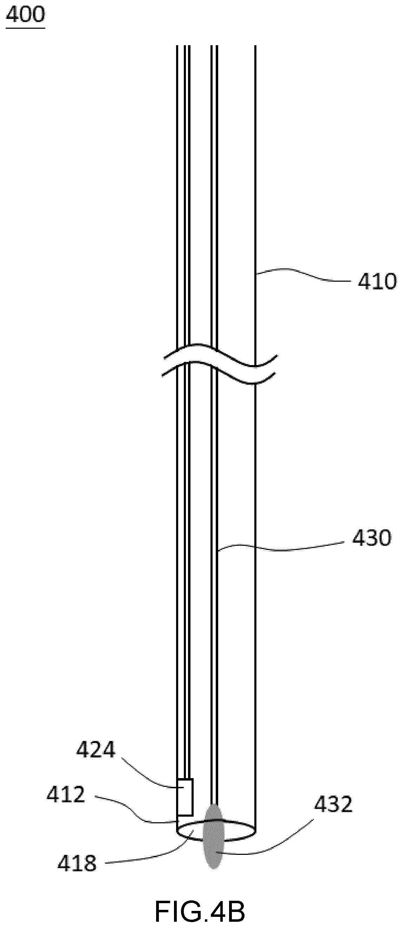

Referring to FIG. 4A and FIG. 4B, there are shown schematic views of the medical endoscope system according to an embodiment of the present disclosure. In the embodiment illustrated in FIG. 4A and FIG. 4B, a medical endoscope system 400 further comprises a shutter device 430, and the shutter device 430 is movably disposed to extend within a sheath device 410 (the description "disposed to extend" means that the shutter device 430 extends within the sheath device 410 along the length direction of the sheath device 410). The user can place the shutter device 430 into the sheath device 410 along the length direction thereof as needed, or can remove the shutter device 430 from the sheath device 410 along the length direction thereof as needed. The shutter device 430 has a shutter portion 432, and the user can shut an opening portion 418 of the sheath device 410 (the opening portion 418 is located at an end portion 412 of the sheath device 410) through the shutter portion 432 while placing the medical endoscope system 400 into the patient's body. In this way, damage to human tissue caused by the opening portion 418 of the sheath device 410 can be prevented. Preferably, the volume of the shutter portion 432 can be controlled by the user. In this way, the volume of the shutter portion 432 can be reduced before moving the shutter device 430 along the sheath device 410; this can help avoid moving the optical sensing device (in particular an optical sensing portion 424 of the optical sensing device) to a wrong position, or avoid damaging the optical sensing portion 424 during the movement of the shutter portion 432. In one embodiment, the shutter portion 432 is an inflatable element, and the user can inflate the shutter portion 432 to increase the volume of the shutter portion 432 as needed (see FIG. 4A), or can deflate the shutter portion 432 to reduce the volume of the shutter portion 432 as needed (see FIG. 4B). Preferably, the shutter portion 432 can be configured to have a specific shape (for example, the shutter portion 432 may have a notch portion 434, and the notch portion 434 will not come into contact with the optical sensing portion 424 when the shutter portion 432 is inflated), thereby ensuring the shutter portion 432 does not press against the optical sensing device (in particular the optical sensing portion 424 of the optical sensing device) when it is inflated. In one embodiment, to simplify the operating process or to lower the manufacturing cost of the shutter device, the shutter portion of the shutter device can have a fixed shape, and can be made of, for example, a plastic material, but is not limited thereto. In one embodiment, since the optical sensing device and the sheath device can be separated from each other as needed (i.e., the optical sensing device is detachably fixed to the sheath device), the shutter portion may not have a notch portion. When using the shutter device, the user does not have to mount the optical sensing device on the sheath device, and thus, the shutter device will not come into contact with or press against the optical sensing device.

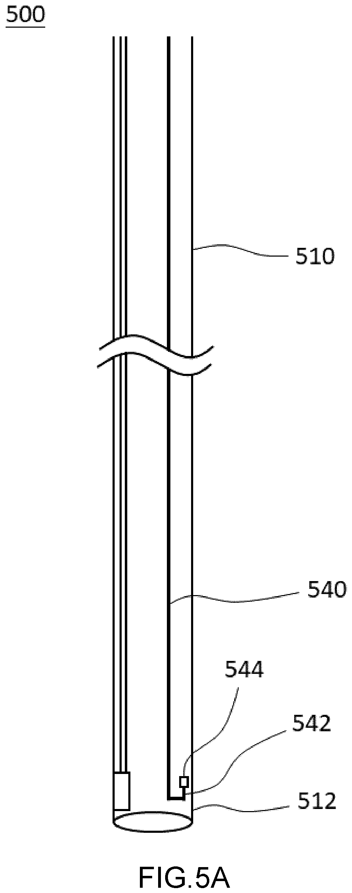

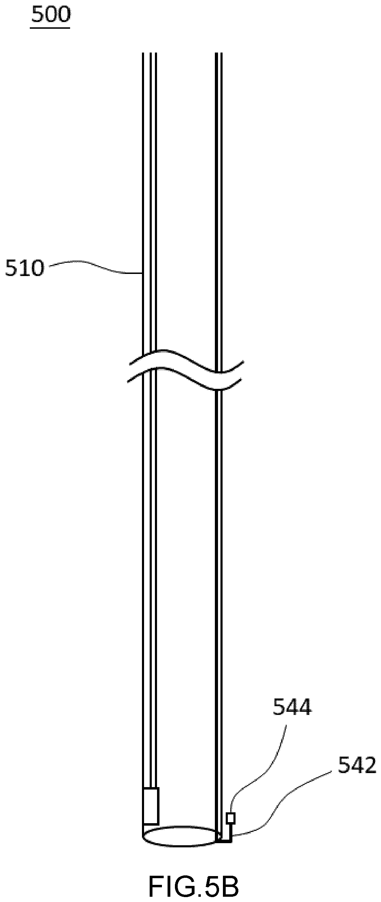

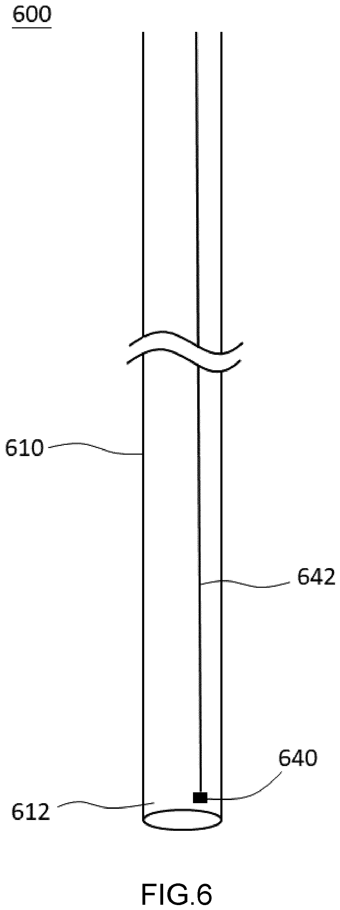

Referring to FIG. 5A and FIG. 5B, there are shown schematic views of the medical endoscope system according to an embodiment of the present disclosure. In the embodiment illustrated in FIG. 5A and FIG. 5B, a medical endoscope system 500 further comprises an electrocautery device 540. The electrocautery device 540 is movably disposed to extend within a sheath device 510 (the description "disposed to extend" means that the electrocautery device 540 extends within the sheath device 510 along the length direction of the sheath device 510 the). The user can place the electrocautery device 540 into the sheath device 510 along the length direction thereof as needed, or can remove the electrocautery device 540 from the sheath device 510 along the length direction thereof as needed. The electrocautery device 540 has a fixing mechanism 542 and an electrocautery portion 544. The electrocautery device 540 is detachably fixed to an end portion 512 of the sheath device 510 through the fixing mechanism 542, and the electrocautery portion 544 is exposed outside the sheath device 510. As a result, the user can use the electrocautery portion 544 of the electrocautery device 540 to perform electrocautery on the target tissue (for example, electrocautery can be applied to target blood vessels or tissues to stop bleeding). In the embodiment illustrated in FIG. 5A and FIG. 5B, the fixing mechanism 542 is a hook-shaped mechanism; the user can have the fixing mechanism 542 fixed to the opening portion of the sheath device 510 by hooking, and have the electrocautery portion 544 exposed outside the sheath device 510. Nevertheless, it should be understood that the fixing mechanism is not limited to a hook-shaped mechanism, and the fixing mechanism may be a mechanism in other shapes as along as it can provide a fixing function. Referring to FIG. 6, there is shown a schematic view of the medical endoscope system according to an embodiment of the present disclosure. In the embodiment illustrated in FIG. 6, a medical endoscope system 600 further comprises an electrocautery device 640, and the electrocautery device 640 is fixed to the outside surface of an end portion 612 of a sheath device 610. The user can use the electrocautery device 640 to perform electrocautery on the target tissue (for example, electrocautery can be applied to target blood vessels or tissues to stop bleeding). Preferably, the electrocautery device 640 is an electrode that realizes electrocautery through a circuit 642 to which power is supplied. It should be understood that when the electrocautery device 640 is composed of electrodes, the number of the electrodes is subject to the user's needs; for example, the electrocautery device 640 may be composed of one electrode, two electrodes (dual electrodes), or three electrodes, but is not limited thereto.

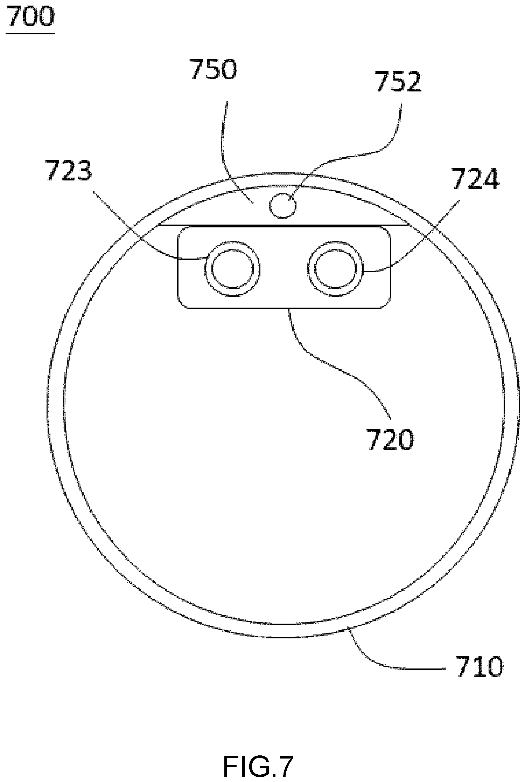

Referring to FIG. 7, there is shown a schematic view of the medical endoscope system according to an embodiment of the present disclosure. In the embodiment illustrated in FIG. 7, a medical endoscope system 700 further comprises a liquid passage portion 750 disposed to extend within a sheath device 710 (the description "disposed to extend" means that the liquid passage portion 750 extends along the length direction of the sheath device 710 within the sheath device 710), and the liquid passage portion 750 has a passage port 752. The passage port 752 of the liquid passage portion 750 is disposed at an end portion of the sheath device 710, and the user can control the medical endoscope system 700 in a way that allows a liquid (for example, water) to flow out of the end portion of the sheath device 710 along the liquid passage portion 750, so as to irrigate the target tissue or wash away a foreign object. In one embodiment, the passage port 752 is disposed close to the optical sensing portions 723, 724 of an optical sensing device 720. Therefore, the user can control the medical endoscope system 700 in a way that allows a liquid (for example, water) to flow along the liquid passage portion 750 to the optical sensing portions 723, 724 of the optical sensing device 720 to irrigate the target tissue.

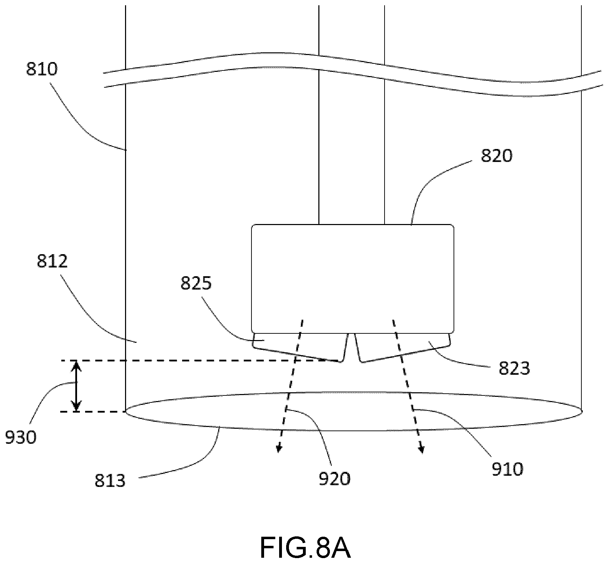

Referring to FIG. 8A, there is shown a schematic view of the medical endoscope system according to an embodiment of the present disclosure. In the embodiment illustrated in FIG. 8A, an optical sensing portion (may be referred to as the "primary optical sensing portion") 820 of the optical sensing device has a first optical sensing portion 823 and a second optical sensing portion 825. The first optical sensing portion 823 is disposed at an end portion 812 of a sheath device 810 and facing a first direction 910 to receive a first optical signal. The second optical sensing portion 825 is disposed at the end portion 812 of the sheath device 810 and facing a second direction 920 to receive a second optical signal. The first direction 910 is not parallel to the second direction 920; thus, the field of view of the first optical sensing portion 823 and the field of view of the second optical sensing portion 825 do not overlap with each other but gradually move apart. In this way, image data from different angles can be obtained, so that images with more different fields of view can be generated. In one embodiment, the end portion 812 of the sheath device 810 comprises an open end portion 813, and there is a retraction distance 930 between the optical sensing portions 823, 825 and the open end portion 813. It should be understood that, depending on the different needs, there may not be a retraction distance between the optical sensing portions 823, 825 and the open end portion 813; instead, the optical sensing portions 823, 825 can be disposed at the open end portion 813, or the optical sensing portions 823, 825 can be disposed slightly protruded from the open end portion 813.

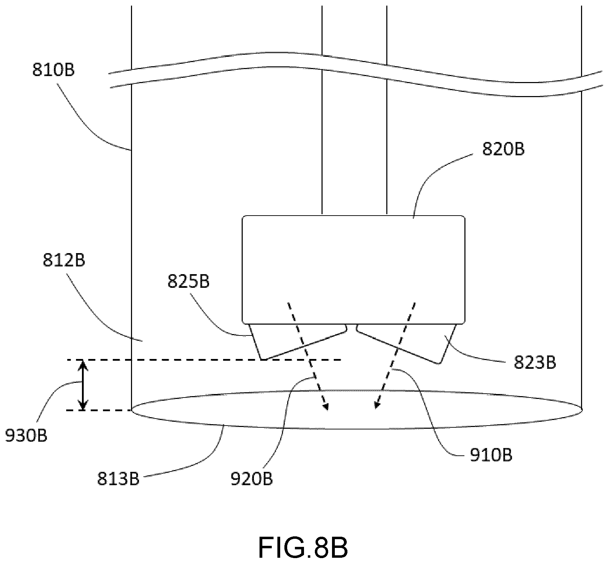

Referring to FIG. 8B, there is shown a schematic view of the medical endoscope system according to an embodiment of the present disclosure. In the embodiment illustrated in FIG. 8B, an optical sensing portion (may be referred to as the "primary optical sensing portion") 820B of the optical sensing device has a first optical sensing portion 823B and a second optical sensing portion 825B. The first optical sensing portion 823B is disposed at an end portion 812B of a sheath device 810B and facing a first direction 910B to receive a first optical signal. The second optical sensing portion 825B is disposed at the end portion 812B of the sheath device 810B and facing a second direction 920B to receive a second optical signal. The first direction 910B is not parallel to the second direction 920B, and the field of view of the first optical sensing portion 823B and the field of view of the second optical sensing portion 825B overlap with each other. In this manner, image data from different angles can be obtained. In one embodiment, a three-dimensional image is obtained through the first optical sensing portion 823B and the second optical sensing portion 825B. In one embodiment, the end portion 812B of the sheath device 810B comprises an open end portion 813B, and there is a retraction distance between the optical sensing portions 823B, 825B and the open end portion 831B. It should be understood that, depending on the different needs, there may not be a retraction distance between the optical sensing portions 823B, 825B and the open end portion 813B; instead, the optical sensing portions 823B, 825B can be disposed at the open end portion 813B, or the optical sensing portions 823B, 825B can be disposed slightly protruded from the open end portion 813B.

The medical endoscope system of the present disclosure is described above and depicted in the drawings. It should be understood that the embodiments of the present disclosure are merely exemplary in nature. Various changes may be made to the embodiments of the present disclosure without departing from the spirit and scope of the claims of the present disclosure, and such changes shall be covered by the scope of the claims of the present disclosure. Therefore, the embodiments of the present disclosure are not intended to restrict the present disclosure, and the actual scope and spirit of the present disclosure is disclosed in the appended claims.

## Claims

1. A medical endoscope system, comprising:
a sheath device having an end portion; and
an optical sensing device disposed to extend within the sheath device and fixed to the end portion,
wherein the optical sensing device has a transmission portion and transmits an optical signal received by the optical sensing device through the transmission portion.

2. The medical endoscope system of claim 1, wherein the end portion of the sheath device is a transparent tubular element.

3. The medical endoscope system of claim 1, further comprising:
a liquid passage portion disposed to extend within the sheath device and having a passage port,
wherein the passage port of the liquid passage portion is disposed at the end portion of the sheath device, and the medical endoscope system allows a liquid to flow out of the end portion along the liquid passage portion.

4. The medical endoscope system of claim 3, wherein the optical sensing device has an optical sensing portion, and the medical endoscope system allows the liquid to flow to the end portion to come into contact with the optical sensing portion.

5. The medical endoscope system of claim 4, wherein the optical sensing portion is a CMOS sensor.

6. The medical endoscope system of claim 5, wherein the optical sensing device has an illumination portion disposed around the optical sensing portion.

7. The medical endoscope system of claim 1, wherein the optical sensing device comprises:
a first optical sensing portion disposed at the end portion and facing a first direction to receive a first optical signal; and
a second optical sensing portion disposed at the end portion and facing a second direction to receive a second optical signal,
wherein the first direction is not parallel to the second direction.

8. The medical endoscope system of claim 1, wherein: the sheath device has a first fixing mechanism disposed at a control end portion of the sheath device;
the optical sensing device has a second fixing mechanism, and the optical sensing device is fixed to the end portion by causing the second fixing mechanism to be connected and fixed to the first fixing mechanism; and
the second fixing mechanism is detachably connected and fixed to the first fixing mechanism.

9. The medical endoscope system of claim 1, wherein the sheath device has a fixing portion disposed at the end portion, and wherein the optical sensing device is detachably fixed to the fixing portion.

10. The medical endoscope system of claim 9, wherein the fixing portion is a magnet.

11. The medical endoscope system of claim 1, wherein the sheath device has a groove disposed to extend on an inner wall surface of the sheath device, wherein the optical sensing portion is connected to the groove and is movable along the groove.

12. The medical endoscope system of claim 1, wherein the end portion comprises an open end portion, and a retraction distance is provided between the optical sensing device and the open end portion.

13. The medical endoscope system of claim 1, further comprising an electrocautery device fixed to an outside surface of the end portion.

14. The medical endoscope system of claim 1, further comprising:
an electrocautery device movably disposed to extend within the sheath device,
wherein the electrocautery device has a fixing mechanism and an electrocautery portion, and the electrocautery device is detachably fixed to the end portion of the sheath device through the fixing mechanism, so that the electrocautery portion is exposed outside the sheath device.

15. The medical endoscope system of claim 1, further comprising:
a shutter device movably disposed to extend within the sheath device,
wherein the shutter device has a shutter portion, and the shutter device shuts an opening portion of the end portion through the shutter portion.

16. The medical endoscope system of claim 15, wherein the shutter portion is an inflatable element.

17. The medical endoscope system of claim 1, further comprising a surgical device disposed within the sheath device.
